Europäisches Patentamt

European Patent Office

Office européen des brevets

(11) Veröffentlichungsnummer: **0 445 800 A1**

## (12) EUROPÄISCHE PATENTANMELDUNG

(21) Anmeldenummer: 91103470.0

(22) Anmeldetag: 07.03.91

(51) Int. Cl.5: **A61N 1/39**, H03K 3/53

(30) Priorität: 07.03.90 DE 4007211

(43) Veröffentlichungstag der Anmeldung:
**11.09.91 Patentblatt 91/37**

(84) Benannte Vertragsstaaten:
**AT BE CH DE DK ES FR GB GR IT LI LU NL SE**

(71) Anmelder: **Müller, Gerhard**
**Amselweg 2**
**W-2361 Wittenborn(DE)**

(72) Erfinder: **Pehrs, Klaus**
**Wacholderweg 38**
**W-2000 Wedel(DE)**

(74) Vertreter: **Vossius & Partner**
**Siebertstrasse 4 P.O. Box 86 07 67**
**W-8000 München 80(DE)**

(54) **Elektrische Schaltung zum Bereitstellen eines Hochspannungsimpulses, inbesondere für einen Defibrillator.**

(57) Es wird eine elektrische Schaltung zur Bereitstellung eines Hochspannungsimpulses, insbesondere für einen Defibrillator, vorgeschlagen, wobei keine Hochspannungsquelle erforderlich ist. Die Hochspannungsimpulse werden in Kondensatoren (C1...Cr) erzeugt, welche durch Schalter (S1...Sn) zur Aufladung parallel zur Entladung jedoch in Reihe geschaltet werden. Die Erfindung gewährleistet eine hohe Sicherheit. Sie gestattet die Verwendung nicht hochspannungsfester, handelsüblicher Bauelemente, den Wegfall galvanisch getrennter Hilfsspannungen und die interne Vernichtung der bereitgestellten Energie ohne zusätzliche Entladungsstromkreise und Lastwiderstände.

Fig. 2

Die Erfindung betrifft eine elektrische Schockelektrodenschaltung, insbesondere für einen Defibrillator.

Defibrillatoren kommen bei der medizinischen Behandlung von Patienten zum Einsatz. Daher werden hohe Ansprüche an die Sicherheit solcher Geräte gestellt, insbesondere die Forderung nach einer möglichst geringen Spannungs- und Strombelastung des Patienten. Desweiteren wird eine individuelle Anpassung der Spannungsimpulskurvenform an den Patienten verlangt. Das geschieht durch zeitlich gestuftes Zuschalten einzelner Spannungsimpulse. Eine im Stand der Technik übliche elektrische Schaltung eines Defibrillators besteht aus mehreren Kondensatoren, die elektrische Energie speichern und über mehrere Schalter entladen werden, um so einen Hochspannungsimpuls für den Defibrillator zur Verfügung zu stellen.

Aus der EP-135735 B1 ist eine elektrische Schockelektrodenschaltung bekannt, bei der mehrere in Reihe geschaltete Kondensatoren in einer bestimmten zeitlichen Reihenfolge entladen werden, um so eine individuell formbare Hochspannungsentladekurve zu erzeugen. Die Aufladung der in Reihe geschalteten Kondensatoren erfordert eine Hochspannung, die bei n Kondensatoren gleich dem n-fachen der von jedem Kondensator abgegebenen Spannung ist. Eine solche Schaltung hat den Nachteil, daß eine Hochspannungsquelle benötigt wird, woraus sich zusätzliche Gewichts- und Isolationsproblemen sowie Gefahren für den Patienten ergeben.

Demgegenüber liegt der Erfindung die Aufgabe zugrunde, eine elektrische Schaltung zum Erzeugen eines Hochspannungsimpulses, insbesondere für einen Defibrillator zur Verfügung zu stellen, wobei keine Hochspannungsquelle erforderlich ist.

Diese Aufgabe wird mit den Merkmalen der Patentansprüche gelöst.

Bei der Lösung geht die Erfindung von dem Grundgedanken aus, die Kondensatoren in Parallelschaltung aufzuladen und in einer für die gewünschte Impulsform geeigneten Reihenschaltung zu entladen.

Die Vorteile der Erfindung bestehen in einer hohen Sicherheit, in der Verwendung nicht hochspannungsfester, handelsüblicher Bauelemente, dem Wegfall galvanisch getrennter Hilfsspannungen und der internen Vernichtung der bereitgestellten Energie ohne zusätzliche Entladestromkreise und Lastwiderstände.

Die Erfindung soll nachstehend anhand der Zeichnungen näher erläutert werden. Es zeigen:

Fig. 1     eine Spannungsimpulskurve für einen Defibrillator nach zeitlich gestuftem Zuschalten von mehreren in Reihe geschalteten Kondensatoren,

Fig. 2     eine erfindungsgemäße elektrische Schaltung.

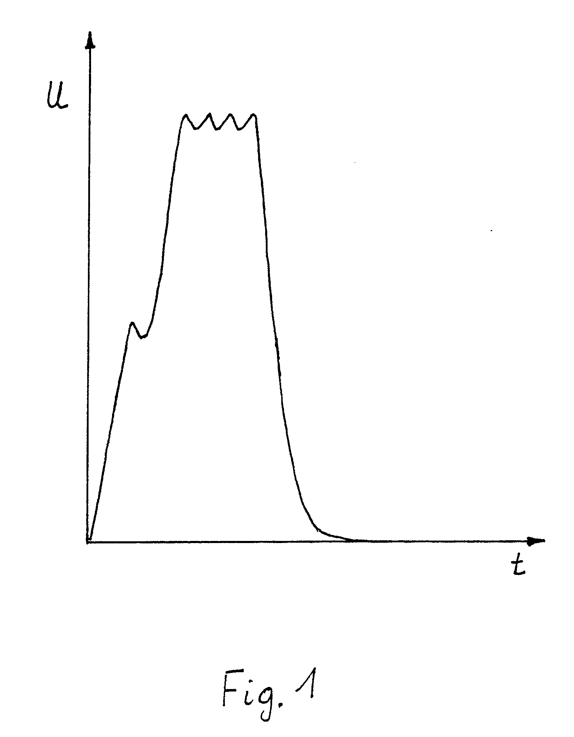

Fig. 1 zeigt eine an einen Patienten individuell anpaßbare Hochspannungsimpulskurve unter Verwendung einer Kondensatorreihenschaltung. Bei n Kondensatoren kann durch wahlweises Zuschalten von minimal je einem bis maximal n Kondensatoren eine gestufte Spannungsimpulskurve über die Zeit erzeugt werden. Dabei werden die Einzelspannungen der gleichzeitig zugeschalteten Kondensatoren zu einem Gesamtspannungsimpuls aufsummiert. Die Form der Spannungsimpulskurve von Fig. 1 ergibt sich auf folgende Weise. Als Anfangsimpuls wird eine bestimmte Anzahl von Kondensatoren mit gleicher Spannung, deren Aufsummierung eine gewünschte Spannung U ergibt, in Reihe zugeschaltet. Dadurch ergibt sich der erste Spannungspeak. Danach werden weitere Kondensatoren in Reihe zugeschaltet, wodurch sich der Spannungsimpuls U z.B. verdoppelt. Durch Zuschalten von Kondensatoren in entsprechend kurzen Zeiten kann ein Spannungsplateau ausgebildet werden.

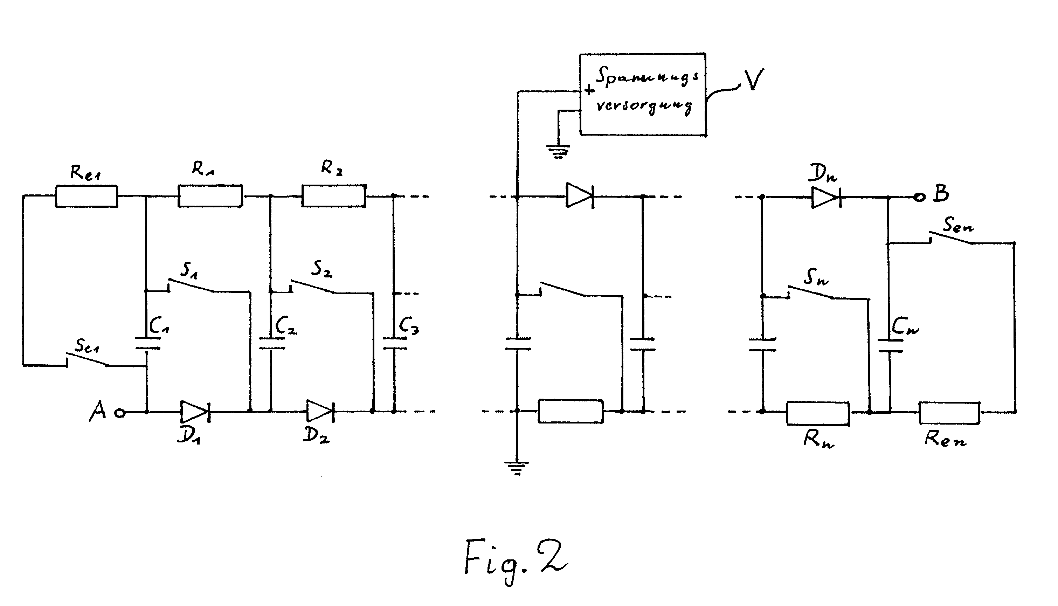

Fig. 2 zeigt eine Schaltung von herkömmlichen Kondensatoren $C_1...C_n$, Schaltern $S_1...S_n$, $S_{e1}$, $S_{en}$, Widerständen $R_1...R_n$, $R_{e1}$, $R_{en}$ und Dioden $D_1...D_n$ mit einer Spannungsversorgung V. Die Kondensatoren $C_1...C_n$ werden bei geöffneten Schaltern $S_1...S_n$, $S_{e1}$, $S_{en}$ zentral über die Dioden $D_1...D_n$ und die Widerstände $R_1...R_n$ aufgeladen. Die dafür erforderliche Spannung ist dabei nur die maximale Spannung eines einzelnen Kondensators, da die Kondensatoren $C_1...C_n$ parallel geschaltet sind. Zur Impulsabgabe werden die Schalter $S_1...S_n$ wahlweise geschlossen, wodurch eine Kondensatorreihenschaltung bewirkt wird. Die resultierende Spannung wird an den Punkten A, B abgenommen. Bei der Reihenschaltung von n Kondensatoren wird eine Hochspannung erzeugt, die dem n-fachen der Spannung eines einzelnen Kondensators und damit dem n-fachen der Spannungsversorgung entspricht. Zum Erzeugen des Hochspannungsimpulses ist also keine Hochspannungsquelle erforderlich. Kondensatoren, die nicht über den Patienten entladen wurden, werden über die Schalter $S_{e1}$, $S_1...S_n$, $S_{en}$ und die Widerstände $R_{e1}$, $R_1...R_n$, $R_{en}$ entladen. Nach Abtrennen der Spannungsversorgung entspricht die Entladezeit, die durch die Widerstände $R_1...R_n$, $R_{e1}$, $R_{en}$ bestimmt wird, annähernd der Ladezeit.

Eine bevorzugte erfindungsgemäße Ausführungsform weist Einrichtungen auf, die die Schaltenergie für die Schalter $S_1...S_n$ aus der im Schaltkreis gespeicherten Energie zur Verfügung stellen.

**Patentansprüche**

1. Elektrische Schaltung zum Erzeugen eines Hochspannungsimpulses mit vorgegebener Signalform, insbesondere für einen Defibrillator,

mit mehreren Kondensatoren ($C_1...C_n$), die zur Erzeugung des Hochspannungsimpulses über mehrere Schalter ($S_1...S_n$) in einer Reihenschaltung wahlweise entladen werden, dadurch gekennzeichnet, daß die Kondensatoren ($C_1...C_n$) in einer Parallelschaltung aufgeladen werden.

2.  Elektrische Schaltung nach Anspruch 1, dadurch gekennzeichnet, daß die Schaltenergie für die Schalter ($S_1...S_n$) der in der Schaltung gespeicherten Energie entnommen wird.

3.  Elektrische Schaltung nach Anspruch 1 oder 2, gekennzeichnet durch eine Steuereinheit zum gesteuerten Zuschalten von aufgeladenen Kondensatoren zu der Kondensatorreihe und Ausbilden eines Spannungsimpulses mit vorgegebenem Signalverlauf.

Fig. 1

Fig. 2

Spannungs versorgung V

| EINSCHLÄGIGE DOKUMENTE | | | EP 91103470.0 |
|---|---|---|---|
| **Kategorie** | **Kennzeichnung des Dokuments mit Angabe, soweit erforderlich, der maßgeblichen Teile** | **Betrifft Anspruch** | **KLASSIFIKATION DER ANMELDUNG (Int Cl⁵)** |
| X | DE - B - 1 234 338 (MINE SAFETY) * Spalte 2, Zeilen 30-35,44-50 * -- | 1 | A 61 N 1/39 H 03 K 3/53 |
| X | GB - A - 1 387 115 (THE LORD MAYOR) * Seite 2, Zeilen 4-12; Fig. * -- | 1,3 | |
| X | US - A - 3 211 154 (BECKER) * Spalte 2, Zeilen 23-27,39-43; Fig. 1,3 * -- | 1,3 | |
| X | US - A - 3 093 136 (LOHR) * Spalte 2, Zeilen 26-28,60-63; Anspruch 1; Fig. 1 * -- | 1,3. | |
| A | EP - A2 - 0 280 526 (INTERMEDICS) * Spalte 12, Zeile 59 - Spalte 14, Zeile 8; Fig. 4, 5 * -- | 1,3 | **RECHERCHIERTE SACHGEBIETE (Int. Cl⁵)** A 61 N H 03 K |
| D,A | EP - A1 - 0 135 735 (GS ELEKTROMED. GERÄTE) * Zusammenfassung; Seite 7, Zeile 6 - Seite 9, Zeile 16; Fig. 5,6 * ---- | 1,3 | |

Der vorliegende Recherchenbericht wurde für alle Patentansprüche erstellt.

| Recherchenort | Abschlußdatum der Recherche | Prüfer |
|---|---|---|
| WIEN | 17-06-1991 | NEGWER |

EPA Form 1503 03 82